# EUROPEAN PATENT APPLICATION

(11) **EP 3 492 105 A1**
(43) Date of publication of application: **05.06.2019**
(21) Application number: 17834195.4
(22) Date of filing: 21.07.2017
(51) Int. Cl.: A61K 45/00, A61K 31/7088, A61K 39/395, A61P 1/02, A61P 43/00

(54) **THERAPEUTIC AGENT FOR PERIODONTITIS AND COMPOSITION FOR TREATING PERIODONTITIS**

(30) Priority: 26.07.2016 JP 2016146593
(71) Applicant: Nihon University, Tokyo 102-8275 (JP)
(72) Inventor: YAMAGUCHI Yoko, Tokyo 102-8275 (JP); OHSHIMA Mitsuhiro, Koriyama-shi Fukushima 963-8611 (JP)
(74) Representative: Regimbeau
(86) International application number: PCT/JP2017/026481
(87) International publication number: WO 2018/021188

(57) **Abstract**

The present invention provides a therapeutic agent for periodontitis including: a hepatocyte growth factor (HGF) signal inhibitor as an active ingredient.

## Description

### Technical Field

The present invention relates to a therapeutic agent for periodontitis and a composition for treating periodontitis. Priority is claimed on Japanese Patent Application No. 2016-146593, filed on July 26, 2016, the content of which is incorporated herein by reference.

### Background Art

Periodontal diseases with which about 80% of adults are considered to be affected with are divided into gingivitis and periodontitis. Gingivitis is a disease caused by bacteria and can be treated. On the other hand, periodontitis is a disease eventually causing teeth to fall out, and there is no effective therapeutic method established.

In the related art, periodontitis is considered to be an infectious disease caused by normal bacterial flora in the oral cavity, and treatment for removing bacteria has been continued. However, about 40% of tooth extraction is caused by periodontitis, and there is still a long way to overcome periodontitis (for example, refer to Non-Patent Document 1).

Collagen is a protein that supports cellular tissues and plays an important role in forming biological tissues such as skin or bones. Collagen is also involved in formation of periodontal tissues. In a case where collagen fibers of tissues of gingiva and periodontal ligament are degraded due to inflammation, connective tissue attachment between the teeth and the alveolar bone is lost and the teeth fall off. The present inventors previously developed a three-dimensional culture system capable of evaluating degradation of collagen in periodontal tissues in vitro under the conditions close to in vivo (refer to Patent Document 1).

### Citation List

### Patent Literature

[Patent Document 1] Japanese Patent No. 5679140

### Non-Patent Literature

[Non-Patent Document 1] Mitsuhiro OSHIMA, Yoko YAMAGUCHI, Paradigm shift in pharmacological treatment of periodontitis, Folia pharmacologica Japonica, 141(6), 314-320, 2013

### Summary of Invention

### Technical Problem

An object of the present invention is to provide a therapeutic agent for periodontitis capable of effectively treating periodontitis.

### Solution to Problem

The present invention is as follows.
(1) A therapeutic agent for periodontitis including: a hepatocyte growth factor (HGF) signal inhibitor as an active ingredient.
(2) The therapeutic agent for periodontitis according to (1), in which the HGF signal inhibitor is selected from the group consisting of an HGF-specific binding substance, an HGF expression inhibitor, an HGF-activating enzyme-specific binding substance, an HGF-activating enzyme expression inhibitor, an HGF-activating enzyme inhibitor, a c-Met-specific binding substance, a c-Met expression inhibitor, and a c-Met inhibitor.
(3) A composition for treating periodontitis including: the therapeutic agent for periodontitis according to (1) or (2); and a pharmaceutically acceptable carrier.

### Advantageous Effects of Invention

According to the present invention, it is possible to provide a therapeutic agent for periodontitis capable of effectively treating periodontitis.

### Brief Description of Drawings

FIGS. 1(a) to 1(d) are views showing a three-dimensional collagen gel culture system of gingival fibroblasts.
FIGS. 2(a) to 2(h) are photographs of each piece of collagen gel incubated in Experimental Example 1.
FIGS. 3(a) and 3(b) are micrographs showing results of hematoxylin-eosin staining of a thin section of collagen gel in Experimental Example 1.
FIG. 4 is a graph showing results of quantitative determination of collagen in Experimental Example 2.
FIGS. 5(a) and 5(b) are photographs of each piece of collagen gel collected after incubation in Experimental Example 3.
FIGS. 6(a) to 6(f) are photographs of each piece of collagen gel incubated in Experimental Example 4.
FIGS. 7(a) and 7(b) are micrographs showing results of hematoxylin-eosin staining of a thin section of collagen gel in Experimental Example 4.

### Description of Embodiments

### [Three-Dimensional Collagen Gel Culture System]

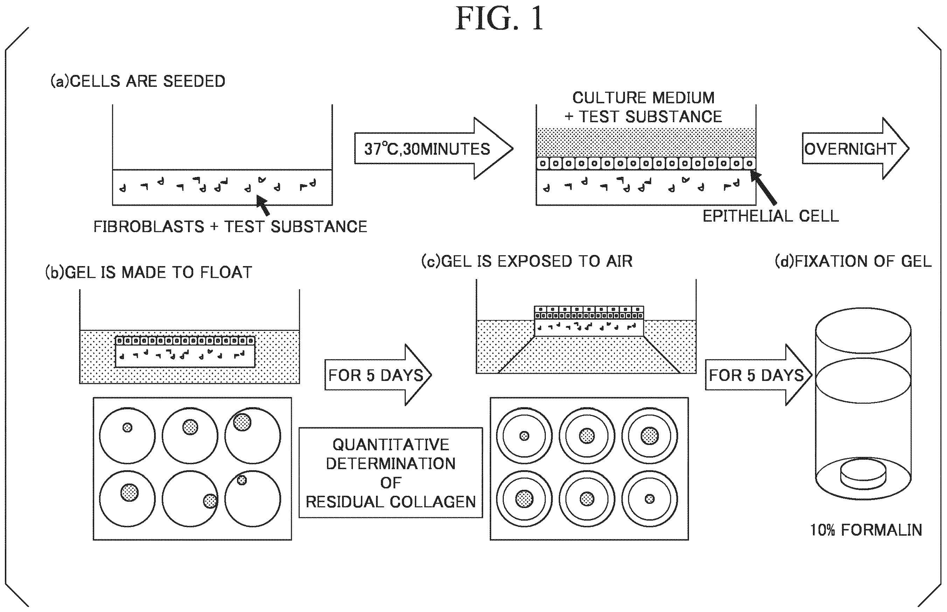

First, a three-dimensional collagen gel culture system of gingival fibroblasts previously developed by the present inventors will be described with reference to FIGS. 1(a) to 1(d). In this culture system, it is possible to evaluate the influence of a test substance on degradation of collagen in periodontal tissues in vitro and to obtain results close to in vivo.

First, as shown in FIG. 1(a), gingival fibroblasts derived from a patient with periodontitis and a test substance are added into collagen gel, and the mixture is poured into a petri dish. Subsequently, the mixture is incubated at 37°C for 30 minutes and the collagen gel is hardened. Subsequently, gingival epithelial cells derived from the patient with periodontitis are seeded on the hardened collagen gel and incubated overnight.

Subsequently, as shown in FIG. 1(b), the collagen gel is peeled off from the petri dish to make the collagen gel float. At this time, the test substance is also added into a medium in which the collagen gel floats. Subsequently, the suspension culture is continued for 5 days. Meanwhile, the collagen gel is degraded and contracts due to various factors produced by an interaction between the gingival fibroblasts and the gingival epithelial cells. In addition, the above-described activity of the gingival fibroblasts and the gingival epithelial cells is affected by the test substance added to the medium, and as a result, the degradation amount of the collagen gel changes.

Subsequently, as shown in FIG. 1(c), the amount of residual collagen is quantitatively determined on day 5 of suspension culture. Alternatively, the surface of the gel may be exposed to air by placing the gel on a cage at this stage to make the gingival epithelial cells multilayered.

Subsequently, the gel is placed in 10% formalin and fixed as shown in FIG. 1(d). A paraffin-embedded thin section of this gel is produced. The produced paraffin-embedded thin section can be subjected to various kinds of staining and can be observed with a microscope.

In this manner, gingival fibroblasts can be three-dimensionally cultured in the collagen gel. In addition, the influence of the test substance on degradation of collagen can be evaluated.

### [Therapeutic Agent for Periodontitis]

The present invention according to an embodiment provides a therapeutic agent for periodontitis containing an HGF signal inhibitor as an active ingredient.

The present inventors performed screening of substances affecting collagen degradation in periodontal tissues of patients with periodontitis using the above-described three-dimensional collagen gel culture system. As a result, as will be described below in examples, it was found that the HGF signal inhibitor remarkably suppresses the degradation of collagen in the three-dimensional collagen gel culture system. This result shows that the HGF signal inhibitor remarkably suppresses the decomposition of collagen in the periodontal tissues of the patients with periodontitis even in vivo.

Therefore, according to the therapeutic agent for periodontitis of the present embodiment, it is possible to effectively treat periodontitis. Currently, no effective therapeutic method for periodontitis has been established. However, according to the therapeutic agent for periodontitis of the present embodiment, it is possible to treat periodontitis with a molecular targeted drug based on a biological rationale.

A signal of HGF is transmitted as follows. First, a proform (single-stranded form) of HGF is activated by a serine protease (HGF-activating enzyme) such as an HGF activator, urokinase, and cathepsin G to form a double-stranded form. Subsequently, HGF is bound to its receptor c-Met to form a dimer of c-Met. Subsequently, a series of signals are transduced with autophosphorylation of tyrosine residue of an intracellular domain of c-Met as a starting point.

The HGF signal inhibitor is not particularly limited, and any HGF signal inhibitor can be used as long as it is a substance blocking the above-described series of signal transduction pathways. Examples of the HGF signal inhibitor include an HGF-specific binding substance, an HGF expression inhibitor, an HGF-activating enzyme-specific binding substance, an HGF-activating enzyme expression inhibitor, an HGF-activating enzyme inhibitor, a c-Met-specific binding substance, a c-Met expression inhibitor, and a c-Met inhibitor.

### (Specific Binding Substance)

Examples of the HGF-specific binding substance include: an HGF specific binding substance which is specifically bonded to the above-described proform of HGF and inhibits activation of HGF with the above-described HGF-activating enzyme; an HGF specific binding substance which is specifically bonded to HGF and inhibits binding of HGF to c-MET; and an HGF specific binding substance which is specifically bonded to HGF and blocks signal transduction to a downstream of c-MET.

In addition, an example of the HGF-activating enzyme-specific binding substance includes an HGF-activating enzyme-specific binding substance which is specifically bonded to the above-described HGF-activating enzyme such as an HGF activator, urokinase, or cathepsin G and inhibits activation of HGF.

In addition, examples of the c-Met-specific binding substance include: a c-Met-specific binding substance which is specifically bonded to c-Met and inhibits binding of HGF to c-MET; and a c-Met-specific binding substance which is specifically bonded to c-Met and blocks signal transmission to a downstream of c-MET.

Here, examples of the specific binding substance include an antibody, an antibody fragment, and an aptamer. The antibody can be prepared, for example, by immunizing an animal such as a mouse with HGF protein, an HGF-activating enzyme, c-Met protein, or a fragment thereof as an antigen. Alternatively the antibody can be prepared, for example, by screening a phage library. Examples of the antibody fragment include Fv, Fab, and scFv. The above-described antibody is preferably a monoclonal antibody. In addition, it may be a commercially available antibody.

The aptamer is a substance having a specific binding ability to a target substance. Examples of the aptamer include a nucleic acid aptamer and a peptide aptamer. A nucleic acid aptamer having a specific binding ability to a target peptide can be selected, for example, through a method of systematic evolution of ligand by exponential enrichment (SELEX). In addition, a peptide aptamer having a specific binding ability to a target peptide can be selected by, for example, a two-hybrid method using yeast.

Specific examples of the HGF-specific binding substance include a humanized antibody (model "AV299", another name "ficlatuzumab", Aveo Oncology Inc.), a human antibody (model "AMG102", another name "rilotumumab", Amgen Inc.), and nucleic acid aptamers disclosed in Japanese Unexamined Patent Application, First Publication No. 2006-149302.

In addition, more specific examples of the HGF-activating enzyme-specific binding substance include anti-HGF activator (HGFA) antibodies disclosed in PCT International Publication No. WO2011/049868. The HGF-activating enzyme-specific binding substance may be a specific binding substance to urokinase, cathepsin G, etc. which activates HGF.

In addition, more specific examples of the c-Met-specific binding substance include nucleic acid aptamers disclosed in PCT International Publication No. WO2012/014890 and a competitive inhibitor of c-Met (model "NK4", Kringle Pharma, Inc.). NK4 is a competitive antagonist to HGF.

### (Expression Inhibitor)

Examples of the expression inhibitor include siRNA, shRNA, miRNA, a ribozyme, an antisense nucleic acid, low molecular compound, a factor inhibiting expression of HGF or c-Met. By administering these expression inhibitors to a living body, expression of HGF, an HGF-activating enzyme, and c-Met can be inhibited. As a result, it is possible to treat periodontitis by inhibiting an HGF signaling.

Small interfering RNA (siRNA) is small-molecule double-stranded RNA of 21 to 23 base pairs used for gene silencing by RNA interference. siRNA introduced into a cell is bonded to a RNA-induced silencing complex (RISC). This complex is bonded to mRNA having a sequence complementary to siRNA to cleave the mRNA. Accordingly, this suppresses gene expression in a sequence-specific manner.

siRNA can be prepared by synthesizing a sense chain/anti-sense chain oligonucleotide using a DNA/RNA automatic synthesizer, for example, by modifying the sense chain/anti-sense chain oligonucleotide in a suitable annealing buffer solution at 90° C to 95°C for about 1 minute and then annealing it at 30°C to 70°C for about 1 to 8 hours.

Short hairpin RNA (shRNA) is a hairpin type RNA sequence used for gene silencing by RNA interference. shRNA may be transferred into a cell by a vector and expressed with a U6 promoter or an H1 promoter, or may be prepared by synthesizing an oligonucleotide having an shRNA sequence with a DNA/RNA automatic synthesizer and performing self-annealing through the same method as that for preparing a siRNA. The hairpin structure of shRNA transferred into a cell is cleaved into siRNA and is bonded to a RNA-induced silencing complex (RISC). This complex is bonded to mRNA having a sequence complementary to siRNA to cleave the mRNA. Accordingly, this suppresses gene expression in a sequence-specific manner.

microRNA (miRNA) is a functional nucleic acid which is encoded on a genome and finally becomes microRNA with about 20 bases while passing through a multi-step formation process. miRNA is classified into functional non-coding RNA (ncRNA: a generic term of RNA that is not translated into protein), and play an important role in a life phenomenon such as regulation of expression of other genes. By administering miRNA having a specific base sequence to a living body, expression of HGF, an HGF-activating enzyme, and c-Met can be inhibited.

A ribozyme is RNA having a catalytic activity. Although ribozymes have various activities, ribozymes aimed at site-specific cleaving of RNAs can be designed by researching the ribozymes as enzymes cleaving RNAs. Ribozymes may be group I intron type ribozymes or M1RNA ribozymes contained in RNase P having a size of greater than or equal to 400 nucleotides, or may be ribozymes, called hammerhead type ribozymes, hairpin type ribozymes, etc. with about 40 nucleotides.

An antisense nucleic acid is a nucleic acid complementary to a target sequence. An antisense nucleic acid can suppress expression of a target gene through inhibition of transcription initiation by formation of triple chains, suppression of transcription by hybridization with a site where a locally open loop structure is formed by RNA polymerase, inhibition of transcription by hybridization with RNA which is in synthesis progress, suppression of splicing by hybridization at a joint point of an intron and an exon, inhibition of splicing by hybridization with a spliceosome forming site, inhibition of transition from nuclei to cytoplasm by hybridization with mRNA, suppression of splicing by hybridization with a capping site or a poly (A) addition site, suppression of translation initiation by hybridization with a translation initiation factor-binding site, suppression of translation by hybridization formation with a ribosome binding site in the vicinity of an initiation codon, inhibition of elongation of peptide chains by hybridization with an mRNA translation region or a polysome binding site, suppression of gene expression by hybridization with an interaction site of nucleic acids and protein, etc.

siRNA, shRNA, miRNA, ribozymes, and antisense nucleic acids may include various kinds of chemical modification in order to improve stability or activity. For example, phosphoric acid residues may be substituted with chemically modified phosphoric acid residues such as phosphorothioate (PS), methyl phosphonate, and phosphorodithionate in order to prevent degradation due to hydrolase such as a nuclease. In addition, at least a part thereof may be constituted by nucleic acid analogues such as peptide nucleic acids (PNAs).

More specific examples of the HGF expression inhibitor include siRNA, shRNA, a ribozyme or an antisense nucleic acid, miR-1991-3p, miR-26a, a valproic acid, and a transforming growth factor (TGF)-β which are specific to HGF.

Examples of the HGF-activating enzyme expression inhibitor include siRNA, shRNA, miRNA, a ribozyme, or an antisense nucleic acid which are specific to an HGF-activating enzyme.

In addition, examples of the c-Met expression inhibitor include siRNA, shRNA, miRNA, a ribozyme, or an antisense nucleic acid which are specific to c-Met.

### (HGF-Activating Enzyme Inhibitor)

The HGF-activating enzyme inhibitor is a substance that inhibits an enzyme that activates a proform (single-stranded form) of HGF to change it into a double-stranded form. Specific examples of the HGF-activating enzyme inhibitor include HGF activator inhibitor type-1, HGF activator inhibitor type-2, and an antibody against an HGF-activating enzyme (for example, anti-HGFA antibody).

### (c-Met Inhibitor)

A c-Met inhibitor is a low molecular compound that blocks transmission of a signal downstream of c-Met. Specific examples thereof include PHA665752 (CAS number: 477575-56-7), Quercetin (CAS number: 117-39-5), MSC2156119J (CAS number: 1100598-30-8), Tivantinib (CAS number: 905854-02-6), XL880 (CAS number: 849217-64-7), PF-02341066 (CAS number: 877399-52-5), and Mao-to (Ephedra Decoction) which is a kind of Kampo medicine.

The HGF signal inhibitor as exemplified above can be used as a therapeutic agent for periodontitis.

### [Composition for Treating Periodontitis]

The present invention according to an embodiment provides a composition for treating periodontitis which contains the above-described therapeutic agent for periodontitis and a pharmaceutically acceptable carrier.

The composition for treating periodontitis of the present embodiment may be formulated in a dosage form to be used orally or a dosage form to be used parenterally. Examples of the dosage form used orally include tablets, capsules, elixirs, and microcapsules. Examples of the dosage form to be used parenterally include injections, ointments, and patches.

A carrier commonly used in a formulation can be used as the pharmaceutically acceptable carrier without particular limitation, and examples thereof include: solvents such as sterilized water and physiological saline; bonding agents such as gelatin, corn starch, tragacanth gum, and gum arabic; excipients such as crystalline cellulose; and bulking agents such as alginic acid.

The composition for treating periodontitis may contain additives. Examples of the additives include lubricants such as magnesium stearate; sweeteners such as sucrose, lactose and saccharin; flavoring agents such as peppermint and akamono oil; stabilizers such as benzyl alcohol and phenol; buffer agents such as phosphate and sodium acetate; solubilizing agents such as benzyl benzoate and benzyl alcohol; antioxidants; preservatives; surfactants; and emulsifiers.

The composition for treating periodontitis can be formulated by appropriately combining additives and the above-described pharmaceutically acceptable carrier and mixing these with each other in a generally acceptable unit dosage form.

Examples of the solvents for injections include an isotonic solution containing adjuvants such as physiological saline, glucose, D-sorbitol, D-mannose, D-mannitol, and sodium chloride. Solvents for injections may contain alcohols such as ethanol, polyalcohols such as propylene glycol and polyethylene glycol; nonionic surfactants such as polysorbate 80 (trademark) and HCO-50.

An example of administration to a patient includes local administration to the gingiva. Examples of preferred dosage forms include injections, ointments, and patches. The dose of the composition for treating periodontitis varies depending on the body weight or age of a patient, an administration method, etc. but those skilled in the art can appropriately select an appropriate dose.

An example of the dose per day in a case where, for example, the dosage form of a composition for treating periodontitis is an injection and a patient is an adult (with a body weight of about 60 kg) include local administration of about 1 µg to 1 mg of an active ingredient (above-described therapeutic agent for periodontitis) per site. The amount of the composition for treating periodontitis described above may be administered at a time or may be administered while being divided into a plurality of times.

### [Other Embodiments]

The present invention according to an embodiment provides a method for treating periodontitis, the method including a step of administering an effective amount of an HGF signal inhibitor to a patient requiring treatment.

The present invention according to an embodiment provides an HGF signal inhibitor for treating periodontitis.

The present invention according to an embodiment provides use of an HGF signal inhibitor for producing a therapeutic agent for periodontitis.

In each of the above-described embodiments, examples of the HGF signal inhibitor include those described above.

### [Examples]

Experimental examples are shown below to describe the present invention in more detail, but the present invention is not limited to the following experimental examples.

### [Experimental Example 1]

An influence of an HGF-neutralizing antibody on degradation of collagen using gingival fibroblasts derived from a patient with periodontitis was examined using a three-dimensional collagen gel culture system.

### (Preparation of Gingival Fibroblasts)

Tissues derived from gingiva excised and became unnecessary during a periodontal surgical operation were cut, and then, the tissue pieces are allowed to stand on the bottom of a cell culture plate. Subsequently, outgrown cells from the tissue pieces were subcultured as a first passage to obtain human gingival fibroblasts.

The obtained gingival fibroblasts were once cultured in the above-described three-dimensional collagen gel culture system to observe collagen resolution, and gingival fibroblasts which extremely decompose collagen gel (that is, with which collagen gel were degraded and contracted). Gingival fibroblasts with which collagen gel having a diameter of 35 mm contracted to a diameter of about 3 mm were selected as the gingival fibroblasts extremely decomposing collagen gel.

In addition, gingival fibroblasts with a low resolution of collagen gel (hereinafter, sometimes referred to as "normal gingival fibroblasts") were used as controls. Gingival fibroblasts with which the contraction of collagen gel having a diameter of 35 mm remained to a diameter of about 10 mm were used as normal gingival fibroblasts.

It has been found from the past studies of the present inventors that gingival fibroblasts which extremely decompose collagen gel are obtained from the gingiva of a patient with periodontitis in a case where the gingival fibroblasts are cultured in the three-dimensional collagen gel culture system. On the other hand, it has been found that gingival fibroblasts which extremely decompose collagen are not obtained from the gingiva of a healthy person.

### (Preparation of Gingival Epithelial Cells)

Gingival tissues excised and became unnecessary during a periodontal surgery were subjected to dispase treatment. Then, epithelial tissues peeled off from a connective tissue portion were cut and the tissue pieces were allowed to stand on the bottom of a plate. Subsequently, cells exogenously generated from the tissue pieces were subcultured as a first generation to obtain human gingival epithelial cells.

### (Preparation of Collagen Gel)

Cell matrix type I-A (Nitta Gelatin Inc.), 5×DMEM, a buffer solution for reconstitution (Nitta gelatin) were mixed with each other to prepare a collagen mixed solution. An HGF-neutralizing antibody (model "AB-294-NA", R&D) was added to this collagen mixed solution so that the final concentration became 20 µg/mL.

In addition, a sample containing no test substance (control) and samples to which HGF (model "100-39", PeproTech, Inc.) was added so that the final concentrations respectively became 25 ng/mL and 50 ng/mL were prepared for comparison.

Subsequently, the above-described gingival fibroblasts which extremely decompose collagen gel were mixed with these collagen mixed solutions and seeded in a 6-well plate so that the cell density became 2.5 × 10⁵ cells/well. Subsequently, the mixture was incubated at 37°C for 30 minutes and the collagen gel was hardened.

Subsequently, the above-described gingival epithelial cells were dispersed by trypsin treatment, and were then seeded on the above-described collagen gel at 2 × 10⁵ cells/well to form a single layer of the epithelial cells.

In addition, the same collagen gel as the above-described collagen gel was produced for comparison except that the above-described normal gingival fibroblasts were used instead of the gingival fibroblasts which extremely decompose collagen gel.

### (Incubation of Collagen Gel)

Subsequently, each prepared collagen gel piece was incubated at 37°C. After 24 hours, each collagen gel piece was peeled off from a plate and was suspended (on day 1 of culture). Each test substance (HGF-neutralizing antibody and HGF) having the same concentration as that described above was added to a medium. Subsequently, suspension culture of collagen gel was continued at 37°C, and collagen gel was observed on day 5 after the start of the suspension culture.

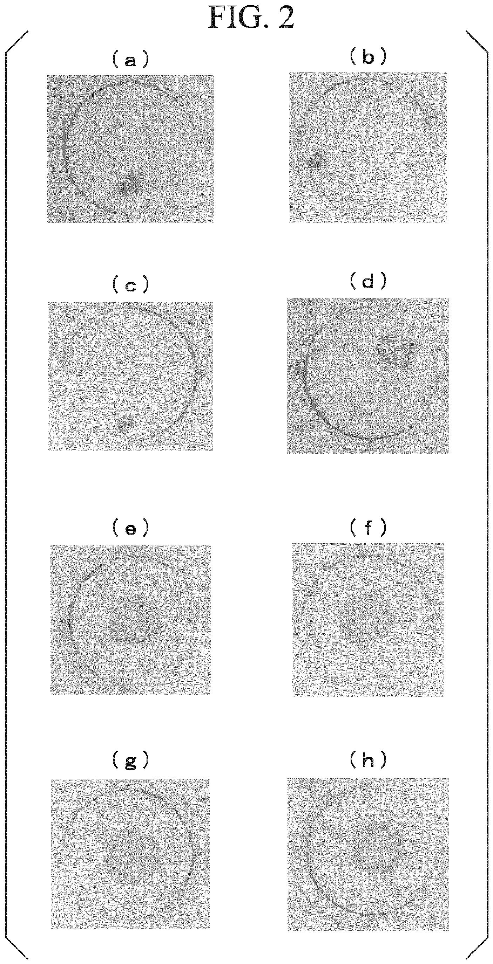

FIGS. 2(a) to 2(h) are photographs of each collagen gel piece on day 5 after the start of the suspension culture. Each mass floating in each well in FIGS. 2(a) to 2(h) is collagen gel. FIGS. 2(a) to 2(d) show results of collagen gel containing gingival fibroblasts which extremely decompose collagen gel. In addition, FIGS. 2(e) to 2(h) show results of collagen gel containing normal gingival fibroblasts.

In addition, FIGS. 2(a) and 2(e) show results of samples containing no test substance (control). FIGS. 2(b) and 2(f) show results of samples to which HGF is added so that the final concentration becomes 25 ng/mL. FIGS. 2(c) and 2(g) show results of samples to which HGF is added so that the final concentration becomes 50 ng/mL. FIGS. 2(d) and 2(h) show results of samples to which HGF-neutralizing antibodies are added so that the final concentration becomes 20 µg/mL.

As a result, it became clear that the degradation of collagen was clearly accelerated compared to the control (FIG. 2(a)) by adding HGF to the collagen gel at a final concentration of 50 ng/mL (FIG. 2(c)).

In addition, it became clear that the decomposition of collagen was remarkably suppressed compared to the control (FIG. 2(a)) by adding an HGF-neutralizing antibody to the collagen gel at a final concentration of 20 µg/mL (FIG. 2(d)). This result shows that the HGF signal inhibitor suppresses the degradation of collagen in the periodontal tissues of patients with periodontitis even in vivo.

On the other hand, there was no difference recognized in any collagen gel of FIGS. 2(e) to 2(h). This result shows that there is no difference in decomposition of collagen even if HGF or an HGF-neutralizing antibody is added to collagen gel containing normal gingival fibroblasts.

### (Microscopic Observation of Thin Section of Collagen Gel)

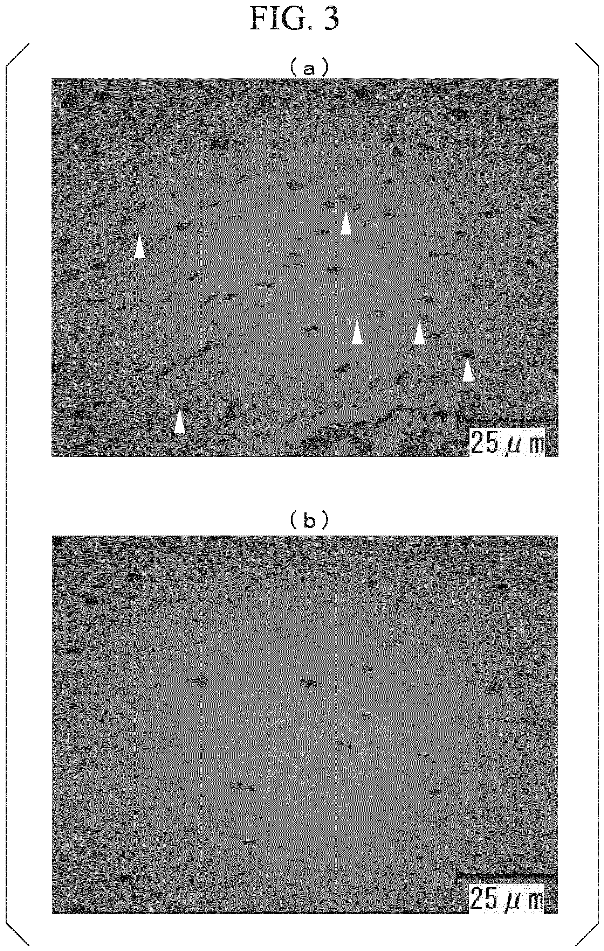

Subsequently, the collagen gel pieces of FIGS. 2(a) and 2(d) were fixed with paraformaldehyde, and then, embedded in paraffin to produce thin section. Subsequently, these thin section were subjected to hematoxylin-eosin staining and observed under a microscope. FIGS. 3(a) and 3(b) are micrographs showing results of hematoxylin-eosin staining. FIG. 3(a) is a photograph showing a result of staining of a thin section of the collagen gel of FIG. 2(a) which is a control. In addition, FIG. 3(b) is a photograph showing a result of staining of a thin section of the collagen gel of FIG. 2(d) to which an HGF-neutralizing antibody is added.

As a result, as shown in FIG. 3(a), a state in which a cell decomposes collagen and a vacuole is formed around the cell was observed in the control collagen gel. In FIG. 3(a), vacuoles are indicated by arrowheads. In contrast, as shown in FIG. 3(b), in the collagen gel to which an HGF-neutralizing antibody was added, vacuoles were hardly recognized around cells. This result further supports that the HGF signal inhibitor suppresses the degradation of collagen in the periodontal tissues of patients with periodontitis.

### [Experimental Example 2]

Effect of an HGF-neutralizing antibody on degradation of collagen using gingival fibroblasts derived from a patient with periodontitis was reviewed using the same three-dimensional collagen gel culture system as in Experimental Example 1. In the present experimental example, residual collagen which had not been degraded was quantitatively determined.

### (Preparation of Collagen Gel)

Cell matrix type I-A (Nitta Gelatin Inc.), 5×DMEM, a buffer solution for reconstitution (Nitta gelatin) were mixed with each other to prepare a collagen mixed solution. An HGF-neutralizing antibody (model "AB-294-NA", R&D) was added to this collagen mixed solution so that the final concentration became 20 µg/mL. In addition, a sample (control) containing no test substance was prepared for comparison.

Subsequently, the same gingival fibroblasts, which extremely decompose collagen gel, as in Experimental Example 1 were mixed with these collagen mixed solutions and seeded in a 6-well plate so that the cell density became 2.5 × 10⁵ cells/well. Subsequently, the mixture was incubated at 37°C for 30 minutes and the collagen gel was hardened.

Subsequently, the same gingival epithelial cells as in Experimental Example 1 were dispersed by trypsin treatment, and were then seeded on the above-described collagen gel at 2 × 10⁵ cells/well to form a single layer of the epithelial cells.

In addition, collagen gel in which normal gingival fibroblasts the same as that in Experimental Example 1 were used instead of the gingival fibroblasts which extremely decompose collagen gel was also produced for comparison.

### (Incubation of Collagen Gel)

Subsequently, each prepared collagen gel piece was incubated at 37°C. After 24 hours, each collagen gel piece was peeled off from a plate and was suspended (on day 1 of culture). HGF-neutralizing antibodies having the same concentration as that described above were added to a medium of a test group. Subsequently, suspension culture of collagen gel was continued at 37°C.

### (Quantitative Determination of Residual Collagen)

Collagen gel was collected on day 5 after the start of the suspension culture, and the amount of residual collagen in the gel was quantitatively determined. The collected gel was solubilized through heat treatment and the collagen was quantitatively determined. A commercially available kit (trade name "Sircol Soluble/Insoluble Collagen Assay Kit", manufactured by Biocolor Ltd.) was used for quantitative determination of collagen.

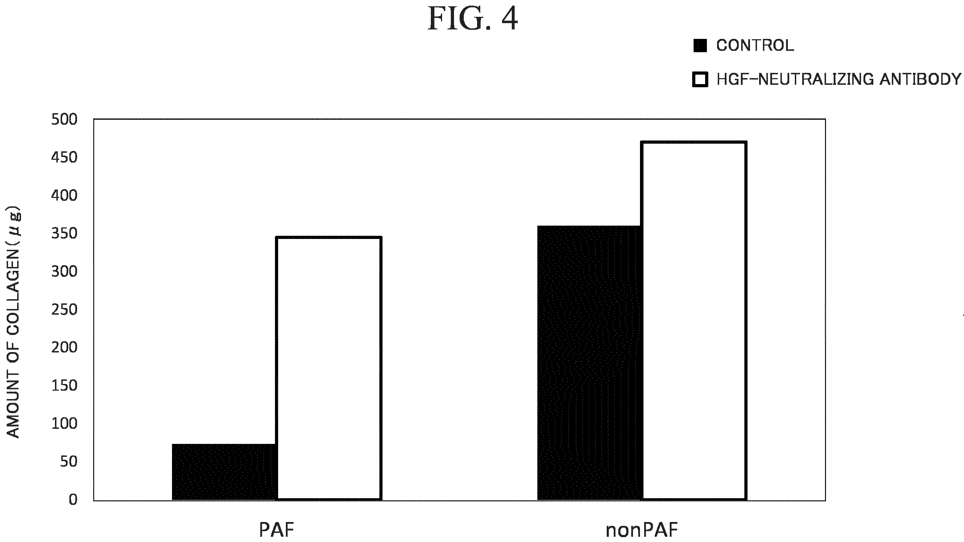

FIG. 4 is a graph showing the amount of quantitatively determined collagen. In FIG. 4, "PAF" shows a result of collagen gel obtained by seeding gingival fibroblasts, which extremely decompose the collagen gel, in the collagen gel, and "nonPAF" shows a result of collagen gel obtained by seeding normal gingival fibroblasts in the collagen gel.

As a result, in the collagen gel in which gingival fibroblasts extremely decomposing the collagen gel, more remarkable degradation was recognized than that in the collagen gel obtained by seeding the normal gingival fibroblasts in the collagen gel. In addition, the decomposition of collagen gel was remarkably suppressed in the collagen gel to which an HGF-neutralizing antibody was added compared to that of the control collagen gel (group in which no HGF-neutralizing antibody was added).

### [Experimental Example 3]

Effect of an HGF-neutralizing antibody on degradation of collagen using gingival fibroblasts derived from a patient with periodontitis was examined through transcriptome analysis, in which a DNA microarray was used, using the same three-dimensional collagen gel culture system as in Experimental Example 1.

### (Preparation of Collagen Gel)

Cell matrix type I-A (Nitta Gelatin Inc.), 5×DMEM, a buffer solution for reconstitution (Nitta gelatin) were mixed with each other to prepare a collagen mixed solution. An HGF-neutralizing antibody (model "AB-294-NA", R&D) was added to this collagen mixed solution so that the final concentration became 20 µg/mL. In addition, a sample containing no test substance (control) and a sample to which HGF (model "100-39", PeproTech, Inc.) was added so that the final concentrations became 50 ng/mL were prepared for comparison.

Subsequently, the same gingival fibroblasts, which extremely decompose collagen gel, as in Experimental Example 1 were mixed with these collagen mixed solutions and seeded in a 6-well plate so that the cell density became 2.5 × 10⁵ cells/well. Subsequently, the mixture was incubated at 37°C for 30 minutes and the collagen gel was hardened. Two kinds of cells (hereinafter, referred to as "PAF1" and "PAF2") were used as gingival fibroblasts extremely decomposing collagen gel.

Subsequently, the same gingival epithelial cells as in Experimental Example 1 were dispersed by trypsin treatment, and were then seeded on the above-described collagen gel at 2 × 10⁵ cells/well to form a single layer of the epithelial cells.

### (Incubation of Collagen Gel)

Subsequently, each prepared collagen gel was incubated at 37°C. After 24 hours, each collagen gel piece was peeled off from a plate and was suspended (on day 1 of culture). HGF-neutralizing antibodies having the same concentration as that described above were added to a medium of a test group. Subsequently, suspension culture of collagen gel was continued at 37°C.

### (Quantitative Determination of Residual Collagen and Preparation of RNA)

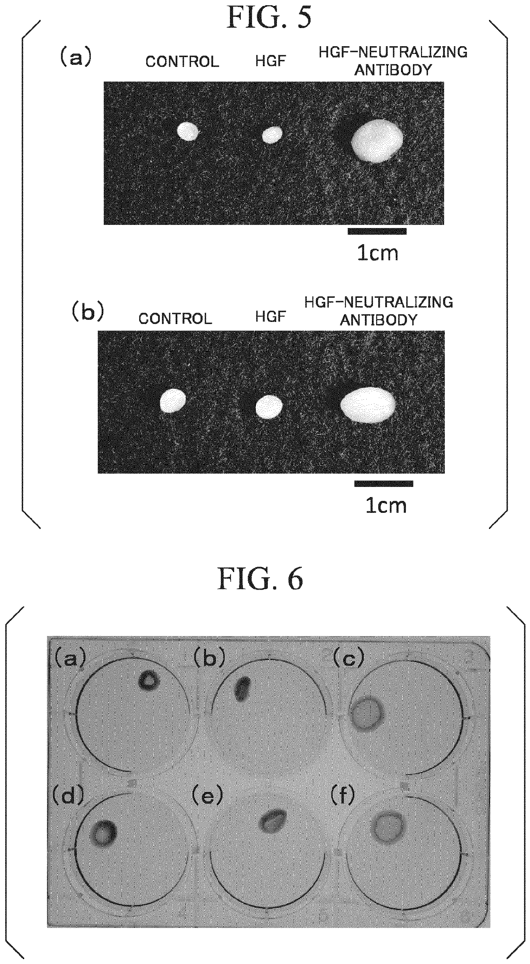

Collagen gel was collected on day 5 after the start of the suspension culture, and the weight thereof was measured. FIGS. 5(a) and 5(b) are photographs of collected collagen gel. FIG. 5(a) shows a result in which the above-described PAF1 was used, and FIG. 5(b) shows a result in which the above-described PAF2 was used. In FIGS. 5(a) and 5(b), the "control" indicates control collagen gel, "HGF" indicates collagen gel to which HGF is added, and the "HGF-neutralizing antibody" indicates collagen gel to which an HGF-neutralizing antibody is added. In addition, Table 1 below shows results obtained by measuring the weight of each collagen gel piece.

**[Table 1]**

| Gingival fibroblasts | Control | HGF addition | HGF-neutralizing antibody |
|---|---|---|---|
| PAF1 | 0.013 g | 0.011 g | 0.151 g |
| PAF2 | 0.034 g | 0.028 g | 0.145 g |

As a result, similarly to the result of Experimental Example 1, degradation of collagen gel was remarkably accelerated in collagen gel to which HGF was added compared to control collagen gel. Furthermore, in collagen gel to which an HGF-neutralizing antibody was added, degradation of the collagen gel was remarkably suppressed. This result further supports that the HGF signal inhibitor suppresses the decomposition of collagen in the periodontal tissues of patients with periodontitis in vivo.

Subsequently, RNA was extracted from each collected collagen gel piece using a commercially available RNA extraction kit (Ambion). Subsequently, the variation in gene expression was analyzed through transcriptome analysis in which a DNA microarray (model "Human Genome U133 Plus 2.0 Array", Affymetrix) was used, using RNA extracted from control collagen gel and RNA extracted from collagen gel to which an HGF-neutralizing antibody was added as samples. Since RNAs were extracted from the entire collagen gel, the analysis was performed in a state in which genes of epithelial cells and fibroblasts coexist. As a result, it became clear that the expression amounts of genes shown in Table 2 are remarkably reduced by adding an HGF-neutralizing antibody to the collagen gel. In addition, although some genes of which expression was increased by the addition of an HGF-neutralizing antibody were recognized, the degree of the expression increase thereof was substantially low.

**[Table 2]**

| Gene symbol | Change in expression amount (Fold Change) |
|---|---|
| IL1A | 0.387 |
| IL1B | 0.549 |
| PLAU | 0.609 |
| KRT6A | 0.624 |
| S100A12 | 0.661 |
| YWHAZ | 0.583 |
| CSTA | 0.653 |
| LAMA3 | 0.475 |
| LAMB3 | 0.556 |
| LAMC2 | 0.401 |
| EMP1 | 0.573 |
| CCND2 | 0.610 |
| CEACAM6 | 0.422 |
| CTSV | 0.239 |
| MMP10 | 0.232 |
| KLK7 | 0.490 |
| HBEGF | 0.366 |
| RIOK3 | 0.477 |
| FGFBP1 | 0.326 |
| MALAT1 | 0.580 |
| AREG | 0.388 |
| SERPINB2 | 0.284 |
| F3 | 0.490 |
| VEGFA | 0.656 |

As shown in Table 2, expression of ILIA and IL1B was remarkably reduced by the addition of an HGF-neutralizing antibody. It is known that ILIA and IL1B induce production of matrix metalloproteases (MMPs) of fibroblasts. In addition, the present inventors have previously revealed that IL-1β or IL-1α derived from gingival epithelial cells enhance collagenase production (mainly MMP-1) of periodontal membranes and gingival fibroblasts. In addition, it is known that both IL-1α and IL-1β induce HGF production of fibroblasts. Accordingly, the fact that the expression of ILIA and IL1B was remarkably reduced by the addition of an HGF-neutralizing antibody further supports that the HGF signal inhibitor is effective as a therapeutic agent for periodontitis.

In addition, as shown in Table 2, expression of PLAU was remarkably reduced by the addition of an HGF-neutralizing antibody. A urokinase-type plasminogen activator (uPA) which is a gene product of PLAU is involved in activation of many MMPs through activation of plasminogen to plasmin. Furthermore, uPA also has a role of converting a proform of HGF with a single-stranded form into one with a double-stranded form. Accordingly, the fact that the expression of PLAU was remarkably reduced by the addition of an HGF-neutralizing antibody further supports that the HGF signal inhibitor is effective as a therapeutic agent for periodontitis.

In addition, as shown in Table 2, expression of cancer-related genes such as various cancer markers or infiltration promoting factors was remarkably reduced by the addition of an HGF-neutralizing antibody. Specifically, expression of KRT6A, S100A12, YWHAZ, CSTA, LAMA3, LAMB3, LAMC2, EMP1, CCND2, CEACAM6, CTSV, MMP10, KLK7, HBEGF, RIOK3, FGFBP1, and MALAT1 was remarkably reduced. The present inventors have previously revealed that periodontitis-associated fibroblasts (PAFs), which are thought to be periodontitis-causing cells highly express genes similar to cancer-associated fibroblasts. Accordingly, the fact that the expression of cancer-related genes was remarkably reduced by the addition of an HGF-neutralizing antibody further supports that the HGF signal inhibitor is effective as a therapeutic agent for periodontitis.

In addition, as shown in Table 2, it became clear that expression of MMP10 and AREG, relating to osteoarthritis or rheumatoid arthritis, was remarkably reduced by the addition of an HGF-neutralizing antibody. In particular, AREG is noteworthy as an exacerbation factor of periodontitis even in association with rheumatoid arthritis. Accordingly, the fact that the expression of AREG was remarkably reduced by the addition of an HGF-neutralizing antibody further supports that the HGF signal inhibitor is effective as a therapeutic agent for periodontitis.

In addition, as shown in Table 2, it became clear that expression of SERPINEB2, F3, and VEGFA, which have been reported in the related art as relating to periodontitis, was remarkably reduced by the addition of an HGF-neutralizing antibody. Among these, FLT1, one of the VEGFA receptors, was previously found by the inventors as periodontitis-causing candidate genes. Accordingly, the fact that the expression of SERPINEB2, F3, and VEGFA was remarkably reduced by the addition of an HGF-neutralizing antibody further supports that the HGF signal inhibitor is effective as a therapeutic agent for periodontitis.

### [Experimental Example 4]

Before clinical application of the therapeutic agent for periodontitis of the present invention to humans, it is necessary to conduct experiments using monkeys. An effect of an HGF-neutralizing antibody on degradation of collagen using gingival fibroblasts derived from a monkey naturally occurred periodontitis was examined using the same three-dimensional collagen gel culture system as in Experimental Example 1. Since it is difficult to culture gingival epithelial cells of monkeys, human gingival epithelial cells were used as gingival epithelial cells.

### (Preparation of Gingival Fibroblasts)

Tissue of a gingiva piece derived from an euthanasia individual of a monkey (Macaca fascicularis, raised in Tsukuba Primate Research Center) naturally occurred periodontitis was cut, and then, the tissue pieces were allowed to stand on the bottom of a cell culture plate. Subsequently, outgrown cells from the tissue pieces were subcultured as a first generation to obtain monkey gingival fibroblasts.

The obtained gingival fibroblasts were once cultured in the above-described three-dimensional collagen gel culture system to observe collagen resolution, and gingival fibroblasts which extremely decompose collagen gel (that is, with which collagen gel were degraded and contracted). Gingival fibroblasts with which collagen gel having a diameter of 35 mm contracted to a diameter of about 3 mm were selected as the gingival fibroblasts extremely decomposing collagen gel.

### (Preparation of Collagen Gel)

Cell matrix type I-A (Nitta Gelatin Inc.), 5×DMEM, a buffer solution for reconstitution (Nitta gelatin) were mixed with each other to prepare a collagen mixed solution. An HGF-neutralizing antibody (model "AB-294-NA", R&D) was added to this collagen mixed solution so that the final concentration became 20 µg/mL. In addition, a sample containing no test substance (control) and a sample to which HGF (model "100-39", PeproTech, Inc.) was added so that the final concentrations became 50 ng/mL were prepared for comparison.

Subsequently, the monkey gingival fibroblasts which extremely decompose collagen gel were mixed with these collagen mixed solutions and seeded in a 6-well plate so that the cell density became 2.5 × 10⁵ cells/well. Subsequently, the mixture was incubated at 37°C for 30 minutes and the collagen gel was hardened.

Subsequently, the gingival epithelial cells prepared in the same manner as in Experimental Example 1 were dispersed by trypsin treatment, and were then seeded on the above-described collagen gel at 2 × 10⁵ cells/well to form a single layer of the epithelial cells. Two kinds of cells were used as human gingival epithelial cells (hereinafter referred to as "SAF1" and "SAF2").

### (Incubation of Collagen Gel)

Subsequently, each prepared collagen gel was incubated at 37°C. After 24 hours, each collagen gel piece was peeled off from a plate and was suspended (on day 1 of culture). HGF-neutralizing antibodies having the same concentration as that described above were added to a medium of a test group. Subsequently, suspension culture of collagen gel was continued at 37°C.

### (Observation of Residual Collagen)

FIGS. 6(a) to 6(f) are photographs of each collagen gel piece on day 5 after the start of the suspension culture. FIGS. 6(a) to 6(c) show results in which the above-described SAF1 was used, and FIGS. 6(d) to 6(f) show results in which the above-described SAF2 was used.

In addition, FIGS. 6(a) and 6(d) show results of samples containing no test substance (control). FIGS. 6(b) and 6(e) show results of samples to which HGF is added so that the final concentration becomes 50 ng/mL. FIGS. 6(c) and 6(f) show results of samples to which an HGF-neutralizing antibody is added so that the final concentration becomes 20 ng/mL.

As a result, it became clear that the degradation of collagen was promoted compared to the controls (FIGS. 6(a) and 6(d)) by adding HGF to the collagen gel pieces at a final concentration of 50 ng/mL (FIGS. 6(b) and 6(e)). Particularly in a case where SAF1 was used, it was recognized that there was a tendency that degradation of collagen was accelerated.

In addition, it became clear that the degradation of collagen was remarkably suppressed compared to the controls (FIGS. 6(a) and 6(d)) by adding an HGF-neutralizing antibody to the collagen gel pieces at a final concentration of 20 µg/mL (FIGS. 6(c) and 6(f)). These results indicate that the HGF signal inhibitor suppresses the degradation of collagen in the periodontal tissues even in a case where gingival fibroblasts derived from a monkey model naturally occurred periodontitis are used, similarly to the case where gingival fibroblasts derived from a patient with periodontitis are used.

### (Microscopic Observation of Thin Section of Collagen Gel)

Subsequently, the collagen gel pieces of FIGS. 6(a) and 6(c) were fixed with paraformaldehyde, and then, paraffin is embedded therein to produce thin sections. Subsequently, these thin sections were subjected to hematoxylin-eosin staining and observed under a microscope.

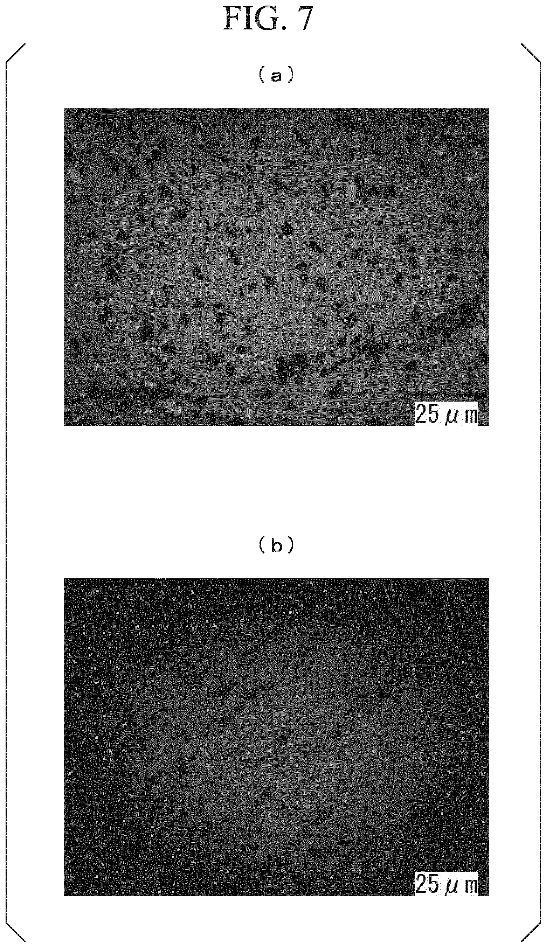

FIGS. 7(a) and 7(b) are micrographs showing results of hematoxylin-eosin staining, and respectively correspond to the collagen gel pieces of FIGS. 6(a) and 6(c). FIG. 7(a) shows a result of a sample (control) containing no test substance and FIG. 7(b) shows a result of a sample to which an HGF-neutralizing antibody is added so that the final concentration becomes 20 µg/mL.

As a result, as shown in FIG. 7(a), a state in which a cell decomposes collagen and a vacuole is formed around the cell was observed in the control collagen gel and the collagen gel to which HGF is added. In contrast, as shown in FIG. 7(b), in the collagen gel to which an HGF-neutralizing antibody was added, vacuoles were hardly recognized around cells.

From the above-described results, it became clear that there are gingival fibroblasts extremely decomposing collagen gel even in the monkey developing periodontitis similarly to the patient with periodontitis. In addition, it became clear that the HGF signal inhibitor suppresses the degradation of collagen in the periodontal tissues even in the case of the monkey similarly to the case of the human.

### Industrial Applicability

According to the present invention, it is possible to provide a therapeutic agent for periodontitis capable of effectively treating periodontitis.

## Claims

1. A therapeutic agent for periodontitis comprising:
a hepatocyte growth factor (HGF) signal inhibitor as an active ingredient.

2. The therapeutic agent for periodontitis according to claim 1,
wherein the HGF signal inhibitor is selected from the group consisting of an HGF-specific binding substance, an HGF expression inhibitor, an HGF-activating enzyme-specific binding substance, an HGF-activating enzyme expression inhibitor, an HGF-activating enzyme inhibitor, a c-Met-specific binding substance, a c-Met expression inhibitor, and a c-Met inhibitor.

3. A composition for treating periodontitis comprising:
the therapeutic agent for periodontitis according to claim 1 or 2; and
a pharmaceutically acceptable carrier.
